# EUROPEAN PATENT SPECIFICATION

(11) **EP 1 882 490 B1**
(45) Date of publication and mention of the grant of the patent: **09.09.2009**
(21) Application number: 05814302.5
(22) Date of filing: 01.12.2005
(51) Int. Cl.: A61M 29/02, A61F 2/01

(54) **DUAL GUIDE WIRE DISTAL PROTECTION DEVICE**
DISTALER SCHUTZFILTER EINES DOPPELTEN FÜHRUNGSDRAHTES
FILTRE DE PROTECTION DISTAL A DOUBLE FIL GUIDE

(30) Priority: 17.05.2005 CN 05241600 U
(43) Date of publication of application: 30.01.2008
(73) Proprietor: Microport Medical (Shanghai) Co., Ltd., 201203 Shanghai (CN)
(72) Inventor: KE, Danian, Pudong New District Shanghai 201203 (CN); XIANG, Yonggang, Pudong New District Shanghai 201203 (CN); LUO, Qiyi, Pudong New District Shanghai 201203 (CN); ZHANG, Yi, Pudong New District Shanghai 201203 (CN); LU, Huijun, Pudong New District Shanghai 201203 (CN); MEI, Qifeng, Pudong New District Shanghai 201203 (CN)
(74) Representative: Feldmeier, Jürgen
(86) International application number: PCT/CN2005/002068
(87) International publication number: WO 2006/122460

(56) References cited:
- WO-A-99/56801
- CN-A- 1 515 326
- CN-Y- 2 643 835
- US-A- 6 142 987
- US-B1- 6 511 492
- US-B1- 6 537 294
- US-B2- 6 527 791

## Description

### Field of the Invention:

This invention relates to a medical device. In particular, it relates to a distal protection device which is suitable for coronary artery in interventional medical treatment.

### Background of the Invention:

In current interventional techniques, there are solid blocks which might drop from the blood vessel during interventional treatment, such as thrombus, scleratheroma debris, etc. Their dropping during interventional surgery will block the downstream blood vessel, thus damaging the downstream organs. For example, if it happens above the neck, it will result in cerebral embolism; if it happens at the heart coronary artery, it will block the artery supplying the apex cordis and result in its necrosis; therefore the heartbeat will locally stop. If a distal protection device is placed at the distal part of the artery (i.e. downstream) during interventional surgery, this risk will be prevented. The function of the protection device is to filter out the block in the blood stream, stopping it from blocking the downstream blood vessel. After the interventional surgery, a filter bag of the protection device and the captured block are retracted into a retractable sheath and taken out of the body.

Currently, the filter bag of the distal protection device is fixed on a guide wire, and contained by a delivery sheath (outer sheath). The guide wire, a protection umbrella and their delivery sheath are sent to the distal end of the pathological changes together. This kind of delivery method is only suitable for peripheral blood vessels such as carotid artery, renal artery etc., in which it is easy for a protection device system to go through the pathological changes of these kinds of blood vessels. But for coronary artery, since the protection umbrella is added on the front end of the guide wire, and the outer sheath covers the protection umbrella, the structure of the whole protection device is comparatively hard and it is difficult for the protection device to go through some thinner, softer and curving coronary arteries. In addition, the protection device is easy to cause damage to or convulsion of the blood vessel, so it is not suitable for heart coronary artery. Therefore, the distal protection device is seldom used for coronary artery interventional medication now, and thus the risk of thrombus block in distal artery during coronary artery interventional operation is increased.

US-6,142,987 A discloses a single guide wire distal protection device having a single guide wire hole. The device comprises a main body of the protection device composed of a self-expanding frame filter bag with holes, a guide wire, and a delivery sheath, wherein the main body of the protection device is fixed to the guide wire by means of a connecting part.

WO 99/56801 A discloses another single guide wire distal protection device.

### Summary of the Invention:

The technical problem addressed by this invention is to design a kind of distal protection device specially used for heart blood vessels with its delivery system which is suitable to pass the heart coronary artery, in order to overcome the above shortcomings of current techniques.

The technical solution of this invention to solve the technical problem is as following:
A dual guide wire distal protection device, including a main body of the protection device composed of a self-expanding frame and a filter bag with holes, a guide wire and a delivery sheath, wherein the main body is fixed to the guide wire by means of a connecting part, there are two guide wire holes on the wall of the delivery sheath, a first guide wire hole and a second guide wire hole, the withdrawn main body of the protection device is fixed between the first guide wire hole and the second guide wire hole by guide wire which passes through the second guide wire hole. After releasing the main body of the protection device, the self-expanding frame is expanded, causing the filter bag covering the frame to expand into umbrella-shape.

Terms: the "distal end" is the end far away from its operating handle, and the "proximal end" is the end close to its operating handle.

The distance from the first guide wire hole to the distal end of the delivery sheath is greater than 50mm and less than the entire length of the delivery sheath, Preferably, the distance is 100∼250mm.

Said delivery sheath further includes an axial guide cannula secured in the delivery sheath, the proximal end of the guide cannula being embedded into the second guide wire hole, the length of the guide cannula being less than the distance between the first guide wire hole and the second guide wire hole.

The delivery sheath is a variable diameter sheath, the diameter of the part in which the guide cannula is located is smaller than that of the distal end of the sheath and that of the nearby region at the distal end of the sheath. The outer wall of the cannula tightly abuts against the inner wall of the delivery sheath, and the main body of the protection device is fixed outside of the distal end of the guide cannula.

There are radiopaque markers at the distal end of said delivery sheath, and there are also radiopaque markers on the self-expanding frame.

Said self-expanding frame is cut from a NiTinol tube, and the material of the filter bag with holes is either PET or PU or PTFE or Nylon.

The use of the device is as following: before use, insert the guide wire of the protection device through the second guide wire hole at the proximal end and pull the guide wire to locate the main body of the protection device between the two guide wire holes. Thus, the sheath distal side hole (i.e. the first side hole) is a piece of empty delivery sheath. The length of this part of empty sheath is 100∼250mm. It can be delivered from the orifice of the coronary to any part of the coronary artery reaching the apex cordis. Before delivery, use a common guide wire to penetrate the pathological part of the coronary artery, and then insert the distal end of the empty sheath of the delivery sheath which contains the protection device from a tail part of the proximal end of the guide wire out of the first side hole, and through the pathological part along the guide wire. There are radiopaque markers at the distal end of the delivery sheath. Locate the radiopaque markers at a position where the filter bag of the protection device is to be released, and take out the common guide wire. Push the main body of the protection device to the end of the sheath by the protection device guide wire fixed with the distal protection device the guide wire of the device, and release the distal protection device by pulling back the delivery sheath. The protection device will get out of the sheath and expand automatically. The radiopaque markers on the main body of the protection device can indicate whether the protection device is released at the desirable position and whether the umbrella expands sufficiently. Take the delivery sheath out of the body after the main body of the protection device is expanded. From the proximal end of the guide wire, the device for intervention is delivered into the pathological region along the wire fixing the main body of the protection device to apply interventional treatment. Take the interventional device out of the body along the guide wire after interventional treatment. Send the retract sheath into the body along the guide wire, to the part for retracting the main body of the protection device. Receive the protection device and the filtered out block into the sheath, take them out of the body simultaneously, and thus finish the whole process of the interventional treatment.

It is known from the above technical solution that the invention has the following advantages compared with the current technology:
1. The invention adopts a dual guide wire structure. The first softer guide wire is used to pass through the pathological region alone, then penetrate the softer empty delivery sheath by means of the first guide wire hole along the first guide wire. Kept inside the soft sheath, the main body of the protection device can penetrate through the pathological region, and is released at the distal part of the target for interventional treatment. In this way, it can be ensured that no coronary damage and convulsion take place. This technique makes it possible for the distal protection device to be used for coronary artery intervention.
2. To adopt the protection device during coronary artery interventional operation will reduce the risk of a downstream artery being blocked;
3. This device can not only be used with stent and balloon intervention, but also be used in traditional thrombus interventional treatment techniques such as thrombus resolving or thrombus removing, thus dramatically improving the safety of thrombus treatment.

It has the advantages of simple structure, convenient operation, good protection effect and so on, especially suitable for heart interventional treatment.

### Brief Description of the Drawings

Fig 1 is a schematic diagram showing the structure of the dual guide wire distal protection device of the present invention before use;
Fig 2 is a schematic diagram showing the structure of the main body of the protection device of the present invention after release;
Fig 3 is a schematic diagram showing the structure of the delivery sheath.

### Detail Description of Embodiment

Hereinafter, the invention will be further described with the help of the drawings.

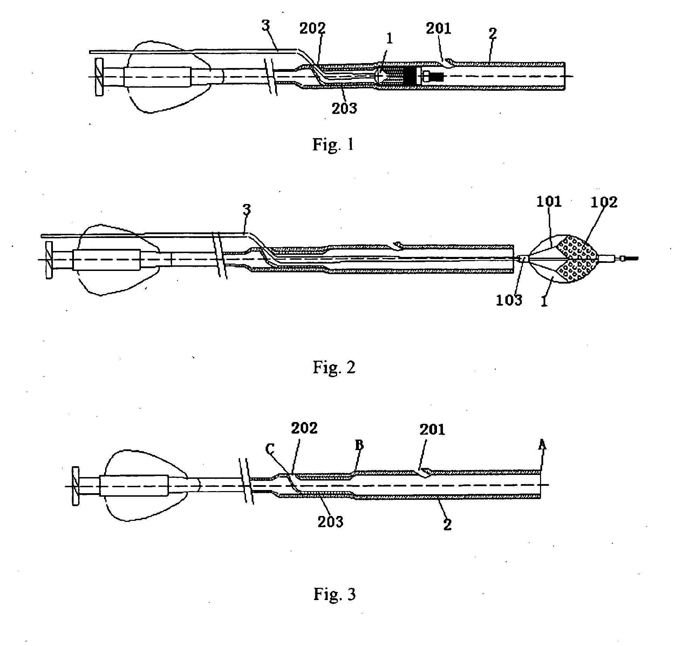

Referring to Fig. 1, Fig. 2 and Fig. 3, the dual guide wire distal protection device of this invention includes a main body 1 of the protection device composed of a self-expanding frame 101 and a filter bag 102 with holes, a protection device guide wire 3 and a delivery sheath 2. The main body 1 of the protection device and the guide wire 3 are fixed with each other through a heavy metal tube 103. There are two guide wire holes on the wall of the delivery sheath 2, a first guide wire hole 201 and a second guide wire hole 202. Said guide wire 3 penetrates through the second guide wire hole 202 and locates the withdrawn main body 1 of the protection device between the first guide wire hole 201 and the second guide wire hole 202.

As a further improvement for this invention, the distance between the first guide wire hole 201 and the distal end A of the delivery sheath is 100∼250mm. The present invention also includes an axial guide cannula 203 which is stabilized in the sheath 2. The proximal end B of the cannula 203 is embedded in the second guide wire hole 202, and the length of the cannula 203 is less than the distance between the first guide wire hole 201 and the second guide wire hole 202. Therefore during the process of withdrawing the main body of the protection device, the guide wire is guided to make it easy to insert the tail of the guide wire of the protection device into the second guide wire hole.

Said delivery sheath 2 is a variable diameter sheath, and the diameter of the part in which the guide cannula is located is less than that of the distal end of the sheath and that of the nearby region at the distal end of the sheath, i.e. the diameter of part AB is greater than that of BC. The outer wall of the cannula tightly abuts against the inner wall of the delivery sheath, and the withdrawn main body 1 of the protection device is located outside of the distal end B of the guide cannula. It is convenient to position the withdrawn protection device inside the sheath by using this variable diameter sheath.

### Example:

Female patient***58 years old, acute heart attack, it is discovered that the A. coronaria dextra is blocked by thrombus. Penetrate the pathological region using the common guide wire first; introduce the delivery sheath of this invention from the distal end of the guide wire, through the first guide wire hole, to the distal end of the pathological region; release the protection device. Perform thrombus resolving treatment by urokinase at the blocked blood vessel. The thrombus is dissolved into small pieces, filtered out by the protection device, and taken out of the body with the retract sheath. The patient is recovered.

## Claims

1. A double guide wire distal protection device, comprising a main body (1) of the protection device composed of a self-expanding frame filter bag (102) with holes, a guide wire (3), and a delivery sheath (2), wherein the main body (1) of the protection device is fixed to the guide wire (3) by means of a connecting part, the double guide wire distal protection device has two guide wire holes (201, 202) on the wall of the delivery sheath (2), a first guide wire hole (201) and a second guide wire hole (202), and the withdrawn main body (1) of the protection device is fixed between the first guide wire hole (201) and the second guide wire hole(202) by the guide wire (3) which passes through the second guide wire hole (202).

2. The double guide wire distal protection device according to claim 1, wherein the distance from the first guide wire hole (201) to the distal end of the delivery sheath (2) is greater than 50mm and less than the entire length of the delivery sheath (2).

3. The double guide wire distal protection device according to claim 1 or 2, wherein the sheath further includes an axial guide cannula (203) secured in the sheath, the proximal end of the cannula (203) being embedded in the second guide wire hole (202), the length of the cannula (203) being less than the distance between the first guide wire hole (201) and the second guide wire hole (202).

4. The double guide wire distal protection device according to claim 3, wherein the delivery sheath (2) is a variable diameter sheath, and the diameter of the part of the delivery sheath (2) in which the guide cannula (203) is located is less than the diameter of the other part from the distal end of the guide cannula (203) to the distal end of the sheath, the outer wall of the guide cannula (203) tightly abuts against the inner wall of the delivery sheath (2), and the main body (1) of the protection device is fixed outside the distal end of the guide cannula (203).

5. The double guide wire distal protection device according to claim 1 or 2, wherein there are radiopaque markers at the distal end of the delivery sheath (2), and there are also radiopaque markers on the self-expanding frame (101).

6. The double guide wire distal protection device according to claim 3, wherein there are radiopaque markers at the distal end of the delivery sheath (2), and there are also radiopaque markers on the self-expanding frame (101).

7. The double guide wire distal protection device according to claim 4, wherein there are radiopaque markers at the distal end of the delivery sheath (2), and there are also radiopaque markers on the self-expanding frame (101).

8. The double guide wire distal protection device according to claim 1 or 2, wherein the self-expanding frame (101) is cut from a Nitinol tube, and the material of the filter bag (102) with holes is selected from PU, PTFE or Nylon.

9. The double guide wire distal protection device according to claim 5, wherein the self-expanding frame (101) is cut from a Nitinol tube, and the material of the filter bag (102) with holes is selected from PU, PTFE or Nylon.

10. The double guide wire distal protection device according to claim 7, wherein the self-expanding frame (101) is cut from a Nitinol tube, and the material of the filter bag (102) with holes is selected from PU, PTFE or Nylon.

## Patentansprüche

1. Doppelführungsdraht-Distalschutzvorrichtung mit einem Hauptkörper (1) der Schutzvorrichtung, der aus einem Selbstentfaltungsrahmen-Filterbeutel (102) mit Löchern, einem Führungsdraht (3) und einer Transporthülse (2) besteht, wobei der Hauptkörper (1) der Schutzvorrichtung an dem Führungsdraht (3) mittels eines Verbindungsteils befestigt ist, wobei die Doppelführungsdraht-Distalschutzvorrichtung zwei Führungsdrahtlöcher (201, 202) in der Wand der Transporthülse (2), nämlich ein erstes Führungsdrahtloch (201) und ein zweites Führungsdrahtloch (202) aufweist, und der herausgezogene Hauptkörper (1) der Schutzvorrichtung zwischen dem ersten Führungsdrahtloch (201) und dem zweiten Führungsdrahtloch (202) durch den Führungsdraht (3) befestigt ist, der durch das zweite Führungsdrahtloch (202) hindurch tritt.

2. Doppelführungsdraht-Distalschutzvorrichtung gemäß Anspruch 1, wobei der Abstand von dem ersten Führungsdrahtloch (201) zu dem distalen Ende der Transporthülse (2) größer als 50 mm und kleiner als die gesamte Länge der Transporthülse (2) ist.

3. Doppelführungsdraht-Distalschutzvorrichtung gemäß Anspruch 1 oder 2, wobei die Hülse des Weiteren eine axiale Führungskanüle (203) aufweist, die in der Hülse gesichert ist, wobei das körpernahe Ende der Kanüle (203) in dem zweiten Führungsdrahtloch (202) eingebettet ist und die Länge der Kanüle (203) kleiner ist als der Abstand zwischen dem ersten Führungsdrahtloch (201) und dem zweiten Führungsdrahtloch (202).

4. Doppelführungsdraht-Distalschutzvorrichtung gemäß Anspruch 3, wobei die Transporthülse (2) eine Hülse mit variablem Durchmesser ist, und der Durchmesser des Teils der Transporthülse (2), in dem sich die Führungskanüle (203) befindet, kleiner ist als der Durchmesser des anderen Teils von dem distalen Ende der Führungskanüle (203) hin zu dem distalen Ende der Hülse, wobei die Außenwand der Führungskanüle (203) dicht an der Innenwand der Transporthülse (2) anliegt, und der Hauptkörper (1) der Schutzvorrichtung außerhalb des distalen Endes der Führungskanüle (203) befestigt ist.

5. Doppelführungsdraht-Distalschutzvorrichtung gemäß Anspruch 1 oder 2, wobei röntgenfähige Marker an dem distalen Ende der Transporthülse (2) vorhanden sind, und wobei röntgenfähige Marker auch an dem Selbstentfaltungsrahmen (101) vorhanden sind.

6. Doppelführungsdraht-Distalschutzvorrichtung gemäß Anspruch 3, wobei röntgenfähige Marker an dem distalen Ende der Transporthülse (2) vorhanden sind, und wobei röntgenfähige Marker auch an dem Selbstentfaltungsrahmen (101) vorhanden sind.

7. Doppelführungsdraht-Distalschutzvorrichtung gemäß Anspruch 4, wobei röntgenfähige Marker an dem distalen Ende der Transporthülse (2) vorhanden sind, und wobei röntgenfähige Marker auch an dem Selbstentfaltungsrahmen (101) vorhanden sind.

8. Doppelführungsdraht-Distalschutzvorrichtung gemäß Anspruch 1 oder 2, wobei der Selbstentfaltungsrahmen (101) aus einer Nitinolröhre geschnitten ist, und wobei das Material des Filterbeutels (102) mit Löchern aus PU, PTFE oder Nylon ausgewählt ist.

9. Doppelführungsdraht-Distalschutzvorrichtung gemäß Anspruch 5, wobei der Selbstentfaltungsrahmen (101) aus einer Nitinolröhre geschnitten ist, und wobei das Material des Filterbeutels (102) mit Löchern aus PU, PTFE oder Nylon ausgewählt ist.

10. Doppelführungsdraht-Distalschutzvorrichtung gemäß Anspruch 7, wobei der Selbstentfaltungsrahmen (101) aus einer Nitinolröhre geschnitten ist, und wobei das Material des Filterbeutels (102) mit Löchern aus PU, PTFE oder Nylon ausgewählt ist.

## Revendications

1. Dispositif de protection distal à double fil guide, comprenant un corps principal (1) du dispositif de protection composé d'un sac filtrant à cadre auto-dilatable (102) avec des orifices, un fil guide (3), et une gaine d'apport (2), dans lequel le corps principal (1) du dispositif de protection est fixé au fil guide (3) au moyen d'une partie de connexion, le dispositif de protection distal à double fil guide possédant deux orifices à fil guide (201, 202) sur la paroi de la gaine d'apport (2), un premier orifice à fil guide (201) et un second orifice à fil guide (202), et le corps principal retiré (1) du dispositif de protection est fixé entre le premier orifice à fil guide (201) et le second orifice à fil guide (202) par le fil guide (3) qui passe à travers le second orifice à fil guide (202).

2. Dispositif de protection distal à double fil guide selon la revendication 1, dans lequel la distance du premier orifice à fil guide (201) à l'extrémité distale de la gaine d'apport (2) est supérieure à 50 mm et inférieure à la longueur entière de la gaine d'apport (2).

3. Dispositif de protection distal à double fil guide selon la revendication 1 ou 2, dans lequel la gaine comprend en outre une canule guide axiale (203) fixée dans la gaine, l'extrémité proximale de la canule (203) étant encastrée dans le second orifice à fil guide (202), la longueur de la canule (203) étant inférieure à la distance entre le premier orifice à fil guide (201) et le second orifice à fil guide (202).

4. Dispositif de protection distal à double fil guide selon la revendication 3, dans lequel la gaine d'apport (2) est une gaine de diamètre variable, et le diamètre de la partie de la gaine d'apport (2) dans laquelle la canule guide (203) est située est inférieur au diamètre de l'autre partie de l'extrémité distale de la canule guide (203) à l'extrémité distale de la gaine, la paroi extérieure de la canule guide (203) prend appui hermétiquement contre la paroi intérieure de la gaine d'apport (2), et le corps principal (1) du dispositif de protection est fixé à l'extérieur de l'extrémité distale de la canule guide (203).

5. Dispositif de protection distal à double fil guide selon la revendication 1 ou 2, dans lequel il y a des marqueurs radio-opaques à l'extrémité distale de la gaine d'apport (2), et il y a également des marqueurs radio-opaques sur le cadre auto-dilatable (101).

6. Dispositif de protection distal à double fil guide selon la revendication 3, dans lequel il y a des marqueurs radio-opaques à l'extrémité distale de la gaine d'apport (2), et il y a également des marqueurs radio-opaques sur le cadre auto-dilatable (101).

7. Dispositif de protection distal à double fil guide selon la revendication 4, dans lequel il y a des marqueurs radio-opaques à l'extrémité distale de la gaine d'apport (2), et il y a également des marqueurs radio-opaques sur le cadre auto-dilatable (101).

8. Dispositif de protection distal à double fil guide selon la revendication 1 ou 2, dans lequel le cadre auto-dilatable (101) est coupé à partir d'un tube en Nitinol, et le matériau du sac filtrant (102) avec des orifices est sélectionné parmi le PU, le PTFE ou le Nylon.

9. Dispositif de protection distal à double fil guide selon la revendication 5, dans lequel le cadre auto-dilatable (101) est coupé à partir d'un tube en Nitinol, et le matériau du sac filtrant (102) avec des orifices est sélectionné parmi le PU, le PTFE ou le Nylon.

10. Dispositif de protection distal à double fil guide selon la revendication 7, dans lequel le cadre auto-dilatable (101) est coupé à partir d'un tube en Nitinol, et le matériau du sac filtrant (102) avec des orifices est sélectionné parmi le PU, le PTFE ou le Nylon.
